(19) Europäisches Patentamt
European Patent Office
Office européen des brevets

(11) **EP 1 940 468 B1**

(12) **EUROPEAN PATENT SPECIFICATION**

(45) Date of publication and mention
of the grant of the patent:
**15.11.2017 Bulletin 2017/46**

(51) Int Cl.:
***A61K 9/08*** (2006.01)   ***A61K 47/36*** (2006.01)

(21) Application number: **06803123.6**

(86) International application number:
**PCT/US2006/034864**

(22) Date of filing: **07.09.2006**

(87) International publication number:
**WO 2007/030623 (15.03.2007 Gazette 2007/11)**

(54) **BI-MODAL HYALURONATE SOLUTION**

BIMODALE HYALURONAT-LÖSUNG

SOLUTION D'HYALURONATE BIMODALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.09.2005 US 715016 P**
**08.09.2005 US 715926 P**

(43) Date of publication of application:
**09.07.2008 Bulletin 2008/28**

(73) Proprietor: **AMO Regional Holdings**
**Quarryvale,**
**Dublin (IE)**

(72) Inventors:
• **BERGMAN, Rolf**
**S-755 98 Uppsala (SE)**
• **LUNDQVIST, Maria**
**S-743 34 Storvreta (SE)**
• **MANNBERG, Stig**
**S-756 47 Uppsala (SE)**
• **LUNDGREN, Bjorn**
**S-757 56 Uppsala (SE)**

• **SHIMIZU, Robert**
**Laguna Niguel, CA 92677 (US)**

(74) Representative: **Hoffmann Eitle**
**Patent- und Rechtsanwälte PartmbB**
**Arabellastraße 30**
**81925 München (DE)**

(56) References cited:
WO-A-93/15744          WO-A-03/059391
WO-A-2006/076485     WO-A1-03/057187
GB-A- 1 340 518         US-A- 4 303 676
US-A- 5 266 563         US-A- 5 492 936
US-A1- 2004 101 561    US-A1- 2004 167 480

• SILVER F H ET AL: "PHYSICAL PROPERTIES OF HYALURONIC ACID AND HYDROXYPROPYLMETHYLCELLULOSE IN SOLUTION: EVALUATION OF COATING ABILITY" JOURNAL OF APPLIED BIOMATERIALS, JOHN WILEY & SONS, INC., NEW YORK, NY, US, vol. 5, no. 1, 1994, pages 89-98, XP008050195 ISSN: 1045-4861

**Description**

**FIELD OF THE INVENTION**

The present invention relates to solutions for use in ophthalmic surgery. More specifically, this invention relates to solutions for use in ophthalmic surgery involving polymers having differing molecular mass for protecting cell layers and tissue exposed to trauma experienced during surgery.

**BACKGROUND**

When the natural lens of the eye becomes hazy or clouded, surgery is often performed to remove the impaired lens. During such surgery, the lens can be broken into a number of different particles and then removed using phacoemulsification. Ophthalmic viscosurgical devices (OVDs) are used to protect intraocular tissues, including the corneal endothelium, and maintain space during the surgical procedures.

The characteristics of OVDs are determined by a number of factors, including the polymer used, the molecular mass of the polymer, and the concentration of the polymer. By way of example, and not of limitation, hyaluronic acid (HA), hydroxyl propyl methyl cellulose (HPMC), chondroitin sulfate (CS), carrageenans and polyacrylamide are all known polymers which have been used to formulate OVDs.

Balazs U.S. Patents 4,141,973 and 4,303,676 teach mono-modal formulations of hyaluronate suitable for ophthalmic use, in which the hyaluronate fraction falls within the range of 750K to 1.2 million, or 1 million to 4.5 million, respectively. Although such formulations are useful, it was later discovered that certain bi-modal formulations with hyaluronate fractions up to 4 million can have superior properties. For example, Francese et al., U.S. patents 6,107,347 and 5,492,936 disclose a bi-modal formulation comprising a first alkali metal and/or alkaline earth metal hyaluronate fraction having a molecular mass in the range of 2 to 4 million, and a second alkali metal and/or alkaline earth metal hyaluronate fraction having a molecular mass of about 200,000 to about 800,000.

While the bi-modal formulations in the Francese patents provide significant benefits over the Balazs formulations, Francese et al. nevertheless failed to appreciate how to manipulate the various fractions to achieve combinations of desired rheological properties.

Rheological properties are influenced by the composition, molecular mass and concentration of the polymers. In the past these three factors have dictated that solutions be formulated in one of two different categories: dispersive or cohesive. Dispersive OVDs are low to medium high viscosity formulations that behave like solutions over the range of shear rates utilized. They can be more difficult to deliver due to the less pronounced shear thinning viscosity and due to the high concentrations that are required for space maintenance. Furthermore, their solution-like properties give dispersive OVDs longer duration in the anterior chamber when surgeons use high flow and increased vacuum. These properties may also make it difficult to remove dispersive OVDs at the end of surgery. If a surgeon fails to remove the OVD sufficiently, it can lead to an increased risk of transient increases in intraocular pressure (IOP).

Cohesive OVDs have higher viscosities at low shear rates, and behave more gel-like at higher shear rates. They can be formulated at lower polymer concentrations, and so can be easier to deliver due to a highly shear thinning viscosity while maintaining space during surgery. Additionally, their cohesive properties at medium to high shear rates make it easier for surgeons to remove these OVDs following surgery. However, under high flow and vacuum settings, there is a risk that a surgeon may prematurely eliminate the cohesive OVD from the anterior chamber.

The problem is that neither dispersive nor cohesive formulations are optimal for all aspects of all surgeries. For example, different viscoelastic characteristics are preferred depending on the intended use of the viscoelastic, as well as the surgical technique of the surgeon. In the case of ophthalmic surgery, for example, those surgeons who prefer faster surgeries with higher flow rates tend to prefer an OVD that retains a comparatively high viscosity under high shear rates. That is, such a surgeon would likely prefer a more cohesive viscoelastic. On the other hand, a surgeon who uses lower flow conditions would be more likely to prefer a more dispersive viscoelastic composition. In the past, some ophthalmic surgeons have compensated for this by using two different viscoelastic agents during the same surgery. Clearly this adds the risk of viscoelastic confusion to a surgery, as well as additional cost and administrative burdens. A single composition that satisfies all of the needs of the surgeon would be preferable.

Some researchers have attempted to address the supposed dichotomy by varying the concentration of the polymers. Others have used a mix of different species of polymers to impart the desired rheological properties. However, there continues to be a need for new formulations that combine both dispersive and cohesive properties. By way of example, and not of limitation, such new formulations are needed in protecting ocular or eye cell layers and tissues exposed to trauma, such as that arising from ophthalmic surgical procedures, and also in protecting other tissues exposed to trauma, such as joints. There is additionally a need for compositions useful for dermatological applications that are not harmful to the body, but which can provide intended results.

## SUMMARY OF THE INVENTION

The present invention provides solutions for use in ophthalmic surgery according to the claims in which a given ophthalmically acceptable solution achieves desirable dispersive and cohesive properties by including non-negligible fractions of compounds having average molecular mass that differ by at least four million. Bi-modal, tri-modal and higher-modal solutions are all contemplated.

In the solution for use in ophthalmic surgery according to the invention, both compounds are a salt of hyaluronic acid.

In other aspects of preferred embodiments, the first fraction has an average molecular mass of at least 4.5 million, more preferably at least 7 million, and most preferably at least 10 million. The second fraction preferably has a molecular mass of less than 1.5 million, in some solutions less than 1 million, and in other solutions between 0.75 and 1 million. These and all other ranges set forth herein include their respective endpoints. In addition, the first and second fractions have average molecular mass that differ by at least 4 million.

In other aspects of preferred embodiments, the first fraction has an average molecular mass of at least 2 million, more preferably at least 5 million, and most preferably at least 10 million. The second fraction preferably has a molecular mass of less than 1.5 million, in some solutions less than 1 million, and in other solutions between 22,000 and 1.5 million. These and all other ranges set forth herein include their respective endpoints. In addition, the first and second fractions have average molecular mass that differ by at least 4 million.

As described in detail herein, a variety of solutions for use in ophthalmic surgery may be prepared, and their properties adjusted based on molecular mass as well as concentration of the various fractions. The concentration of polymer in solutions for use in ophthalmic surgery prepared according to the present invention typically ranges from about 10 to about 80 mg/ml, or from about 15 to about 60 mg/ml, or from about 20 to about 30 mg/ml. Knowing that the relative concentrations of the individual fractions may be determined based on the desired properties of the solution as well as the molecular mass of the fractions being used, one of ordinary skill in the art will be able to determine appropriate relative concentrations for the individual fractions.

In still other aspects, the important dispersive and cohesive properties of viscoelastics can be controlled by the percentages and concentrations of the fractions. These characteristics will then give the solution different properties dependent on the magnitude of the shear forces in action.

In one aspect of the invention, different molecular mass fractions of the same polymer are utilized. Since these fractions will have the same refractive index, there is no issue of optical clarity. In other aspects of the invention the different molecular mass fractions comprise different polymers.

The ability to modulate the properties of viscoelastic solutions for use in ophthalmic surgery according to the present invention has potential benefits in ophthalmic surgery. Other indications which form part of the present disclosure and for which this ability has potential benefits are orthopedic uses, dermal/cosmetic uses and modulation of wound healing. The manipulation of rheological and other physical characteristics can provide distinct advantages in performing tissue and medical device manipulations in surgery (eg, manipulation of IOLs and control of tissue during surgery). In addition, these properties can be used to modulate viscoelastic removal (in the case of cataract surgery) or improve viscoelastic retention (in the cases of dermal fillers and orthopedic supplements). By way of example, and not of limitation, examples of ophthalmic surgery may include cataract and glaucoma surgery. By way of example, and not of limitation, orthopedic uses may include synovial injections. Further by way of example, and not of limitation, dermal and cosmetic uses may include dermal fillers.

Any polymer used in a solution for use in ophthalmic surgery according to the present invention can be derived from any source that is known in the art, providing that the source can provide material having the purity that is required for the specific application to which the present invention is being used (e.g., medical grade). In some embodiments of the invention, combinations of polymers may be utilized. As detailed herein, many different polymers will be suitable so long as they are (a) biocompatible and (b) internally compatible with one another in solution.

## BRIEF DESCRIPTION OF THE DRAWINGS

Many aspects of the invention can be better understood with reference to the following drawings:

Figure 1 is a graph showing rheological properties of three monomodal solutions of sodium hyaluronate having a mol. mass of 4,300,000, the solutions varying in concentration from 2 mg/ml to 10 mg/ml;

Figure 2 is a graph showing rheological properties of five monomodal solutions of sodium hyaluronate of varying molecular mass, but constant concentration;

Figure 3 is a graph demonstrating the ability to construct a solution having substantially any desired apparent

molecular mass ($M_{app}$) by changing the proportions and the average molecular mass of each fraction;

**Figure 4** is a plot of the molecular mass distribution of a bimodal formulation as determined using GPC chromatography coupled with Low Angle Light Scattering (LALLS) detection;

**Figure 5** is a graph showing the shear rates of various solutions according to the present invention. The solutions D-002, D-003 and D-006 are not according to the invention. D-007, D-008, D-009 and D-010 are also not according to the invention and are provided for information only.

**Figure 6** is a graph showing viscosity as a function of shear rate for four different formulations. Healon is not according to the invention and is provided for information only.

**Figure 7** is a graph showing the zero shear rate and viscosity under flow for two different bi-modal formulations; and

**Figure 8** is a graph showing the zero shear rate and viscosity under flow for two different bi-modal formulations.

## DETAILED DESCRIPTION

Viscoelastic (VE) formulations can be characterized by a number of different physical parameters. The parameters of greatest interest herein are those judged to be important for explaining / predicting the function of the OVD in different steps during eye surgery, including the following:

1. Rheology
2. Molecular mass
3. Concentration
4. Osmolality
5. Dynamic contact angle
6. Distribution of molecular masses

## 1. Rheology

Rheology is the science of deformation and flow of matter. A number of important parameters for the VE solutions can be revealed by performing various tests using a cone-plate rheometer.

**Shear Viscosity Test.** In one such test a VE-solution is put on a plate, and a cone having a defined geometry (diameter and cone angle) is lowered down into the solution, but still providing a specified gap between the cone and the plate. Depending on what features will be investigated, a torque (shear stress) is then applied to the cone and the resulting strain is recorded. The torque can be applied using either a constant (rotation) or oscillating motion. Depending on the proportion of viscous and elastic properties of the solution being tested, the applied shear stress will partly be lost as frictional heat or partly stored.

To design VE solutions to meet the different flows and shear stresses that will occur during eye surgery, it is important to have knowledge about its resistance to flow at different shear rates. That response is found in a viscosity versus shear rate curve. These measurements are performed in a rheometer with, for example, a cone-plate arrangement. The measurements of the viscosity are performed at several different but constant shear rates (constant angular velocity of the cone relative to the plate). From the resulting curves [log(viscosity) vs log (shear rate)] the zero shear viscosity (ZS) is extrapolated from the plateau region where the viscosity no longer changes at lower shear rates. To achieve reliable results the rheometer must be able to monitor viscosities at shear rates below 1/1000 $s^{-1}$.

There are three points in the shear viscosity curve that can be used as a rough estimate for the shape of the curve. The viscosity at higher shear rates, 1000 $s^{-1}$, is considered to be roughly equivalent to that mostly associated with injection and removal of the VE solution. The viscosity at shear rate 1 $s^{-1}$ is generally considered to be where movement of surgical tools in the eye and unfolding of the IOLs are generally considered to take place. The zero shear viscosity, extrapolated from the flat part of the shear viscosity curve at lower shear rates, shows the viscosity of the solution at rest.

To confirm that the intended measurements will be performed within the linear viscoelastic range (LVE), researchers usually perform an amplitude sweep before making any rheological measurements. Within this range all imposed deformations should be reversibly recovered, except where frictional heat is lost and the resistance (modulus for shear viscosity) should be constant.

**Oscillation Test.** In contrast with the "shear viscosity" test performed under constant rotation, there is another useful test based upon oscillation. In the oscillation test the rheometer is programmed to oscillate the measuring cone at a constant frequency, but with increasing amplitude until the modulus of elasticity at shear G' and the modulus of viscosity at shear G" show that the "structure" in the VE solutions is broken. The elastic modulus G' usually first signals that the structure in the solution is broken and the point where this starts to happen is recorded. Both the value of the modulus before break and the degree of deformation at that point are recorded.

Thus the amplitude sweep gives information about the "net work strength" G', in the LVE, denoted **A(Pa),** the degree of deformation at break of solution structure, **A(%),** and the difference, **G'-G"**, between the modules of elasticity and viscosity at shear.

**Frequency Sweep.** Frequency sweeps are performed in the LVE region at a constant and "safe" amplitude, determined in the amplitude sweep. The oscillating frequency for the rheometer cone typically starts at a frequency below 0.01 Hz and goes up to 100 Hz. The main interest is focused on the cross over between the modules of elasticity and viscosity at shear. The frequency at cross over ($\omega_{co}$) is correlated to the elasticity of the solution and thus also to the molecular mass of the polymers. At this frequency the modules are equal (G'=G"). A low value of ($\omega_{co}$) indicates that the solution is more elastic than that showing a cross over frequency at a higher value.

## 2. Molecular Mass

The mass average relative molecular mass ($M_{rm}$) of a test solution can be determined from low angular laser light scattering (LALLS) experiments. The light scattering intensity at a low scattering angle ($\sim 6°$) is recorded for a series of different degrees of dilution for each formulation. One of ordinary skill in the art will realize that, depending on the equipment and testing protocol, in other testing scenarios the scattering angle may vary from 6°. By way of example, and not of limitation, the angle may vary from 3-8°. A least square fit to the values is extrapolated to zero concentration from which the mass average molecular mass is calculated.

By definition, the mass average relative molecular mass $M_{rm}$ for a typical, mono-modal solution, is expressed as

$$M_{rm} = \frac{\sum n_i \times M_i^2}{\sum n_i \times M_i} \qquad (1)$$

where $n_i$ is the number of molecules with mass $M_i$.

If (1) is applied to a bi-modal or multi-modal formulation to calculate the $M_{rm}$, which hereafter will be noted as $M_{app}$, to show that it is the mass average molecular mass of a multi-modal formulation. $M_{app}$ can be expressed as

$$M_{app} = \frac{\sum X_i \times M_{rm(i)}^2}{\sum X_i \times M_{rm(i)}} \qquad (2)$$

where $M_{rm(i)}$ now correspond to the distribution averages $M_{rm}$ of the $i^{th}$ HA-substance lot in proportions $X_i$ bi/multi-modal formulation.

## 3. Concentration

Concentration of the polymer in the viscoelastic solution may be determined by any means known in the art. For example, in the case of HA, concentration is preferably determined indirectly, by treating a sample with concentrated sulphuric acid. Under those conditions, sodium hyaluronate is converted into glucuronic acid, which gives a color with carbazole. The intensity of the color is recorded at 530 nm with a spectrophotometer, and the concentration of sodium hyaluronate can be calculated from the intensity.

## 4. Osmolality

Osmolality can be calculated from measurements of the freezing point depression. This result is obtained because the value for osmolality has little or nothing to do with the concentration of the polymers. For example, when HA is examined, the result depends mostly on the ions present in the buffer solution, and the number of counter ions ($Na^+$) carried by the carboxylic groups on the HA. By determining freezing point depression, one can therefore estimate osmolality and then the amount of HA present. All investigated formulations in our study were shown to have osmolalities between (200-400) mOsm/kg, which is the interval recommended in the ISO 15978:2001, the OVD standard.

## 5. Dynamic Contact Angle

Contact angles on cleaned hydrophilic microscope glass slides were measured as a function of time. A drop of HA solution is put onto the glass slide, and the drop is analyzed by a digital camera and computer software. The computer performs a best fit to the left and right contact angles of the drop, and calculates their mean value.

The end points of this dynamic process were taken as the value of the contact angle at 90 seconds, ($\theta_{90}$). and the slope defined as the difference between the contact angle at ($\theta_{90}$- $\theta_{190}$) (90-190). This specific time interval was chosen due to a high signal to noise ratio for all formulations.

## 6. Distribution Of Molecular Masses

This ion exchange HPLC method has so far been used to show whether, and to what degree the chosen HA components have overlapping distributions. However, as one of ordinary skill in the art will know, either HPLC and GPC coupled with LALLS may alternatively be used.

The final solutions for use in ophthalmic surgery are relatively homogeneous, and the preparation of the polymer solutions can be done by any means that is known in the art. By way of example, and not of limitation, samples according to the present invention can be prepared in accordance with the following method.

First, the amount of different polymers required based on molecular mass and final solution properties is calculated. The polymers are then placed in an empty vessel to which a buffer solution is also added. This solution is mixed for several hours until it is homogeneous. The solution is then filled into the primary package (i.e., glass cylinders or syringes) and sterilized. One method of sterilization is steam sterilization.

Bi-modal or multi-modal solutions prepared in accordance with the above method can be further treated by means known in the art to remove any air bubbles. By way of example, and not of limitation, the solutions can be centrifuged to remove air bubbles. The solution may alternatively or additionally be sterilized by means known in the art. By way of example, and not of limitation, the solutions can be autoclaved for sterility.

The present ophthalmically acceptable solutions for use in ophthalmic surgery are preferably essentially free of protein and essentially non-pyrogenic. The present solutions for use in ophthalmic surgery preferably include less than about 0.5% by weight of protein based on the total weight of first and second alkali/alkaline earth metal hyaluronates. More preferably, the present solutions for use in ophthalmic surgery have no detectable protein content and no detectable pyrogenicity.

The present solutions for use in ophthalmic surgery include a liquid solvent. In one embodiment of the invention, the solvent is water. The present ophthalmically acceptable solutions for use in ophthalmic surgery are sterile since they are designed for use in the eye.

The present solutions for use in ophthalmic surgery may also include one or more of the following: a buffer and tonicity carnponent(s). When a buffer is used, it is in an amount effective to control the pH of the solution, and to maintain the pH in a range that is acceptable in the setting in which the present invention is being used. By way of example, when the present invention is used as an OVD in the eye, the pH of the present invention is in the range of from about 6 to about 8, and preferably from about 7 to about 7.5. When a tonicity component (or components) is used, they are preferably used in amounts effective to control the osmolality of the solution so that it is suitable in the setting in which the present invention is being used. By way of example, when the present invention is used as an OVD in the eye, the osmolality of the present invention is in the range of from about 200 to about 400, and preferably from about 250 to about 350 mOsmol/kg.

Inclusion of the buffer and/or the tonicity component(s) is particularly useful.

As noted above, when the solution is buffered, the pH is generally between 6.5 and 8, and preferably between 7.0 and 7.5. The tonicity adjustor component(s) act to enhance the compatibility between the solution, and the corneal tissue and/or avoid damage to the corneal tissue. The solution is preferably isotonic.

Examples of suitable buffers are well known in the art and include, but are not limited to, acetate buffers, citrate buffers, phosphate buffers, borate buffers and the like (and mixtures thereof). Examples of suitable tonicity agents include, but are not limited to, alkaline and alkaline earth metal salts. By way of example, and not of limitation, sodium chloride and/or potassium chloride can be used.

The present solutions for use in ophthalmic surgery may include one or more components in amounts effective to provide one or more useful properties and/or benefits to the present solutions for use in ophthalmic surgery.

When the present invention is used as an OVD during an ophthalmic surgical procedure, it is particularly suited to protect the human or animal ocular or eye cell layers and tissues which are being exposed to trauma. By way of example, the present invention is suitable for use during ophthalmic surgery when the natural lens is broken into a number of pieces prior to its removal. In one embodiment, the present invention is a solution for use in ophthalmic surgery, wherein the surgery comprises administering a protective amount of the solution for use in ophthalmic surgery to the ocular or eye cell layers and tissues which are subject to exposure to trauma prior to exposure to the trauma. The solutions for use in ophthalmic surgery of the present invention will adhere to, or at least partially coat, the cell layers and tissues and/or provide protection for the cell layers and/or tissues from the trauma imposed by the surgery.

## Uses

One example of a surgical procedure for which the solution for use in ophthalmic surgery of the present invention is

particularly suited is the method by which the natural lens is removed from an eye. In such a method, a protective amount of a solution for use in ophthalmic surgery according to the present invention is introduced, such as by injection, into the eye. Such introduction can be through an incision formed in the eye, and can be made by any means known in the art including, for example, a cannula or the like. The solution for use in ophthalmic surgery preferably adheres to at least a portion of the eye cell layers and tissues, in particular the corneal endothelium, while allowing sufficient space maintenance such that the surgeon can perform the required steps of the procedure. Once a protective amount of the solution for use in ophthalmic surgery of the present invention is in place, the surgeon may then remove the natural lens. The removal of the lens can be through any means known in the art including, but not limited to, phacoemulsification and associated irrigation/aspiration procedure. Without an OVD, for example an OVD solution for use in ophthalmic surgery according to the present invention, the portions of the eye in proximity to the natural lens (that are subjected to trauma associated with the phacoemulsification) can be damaged.

When desired by the surgeon, the solution for use in ophthalmic surgery of the present invention can be removed from the eye using irrigation/aspiration. The removal is comparatively gentle, and may occur without adversely affecting the remaining portions of the eye.

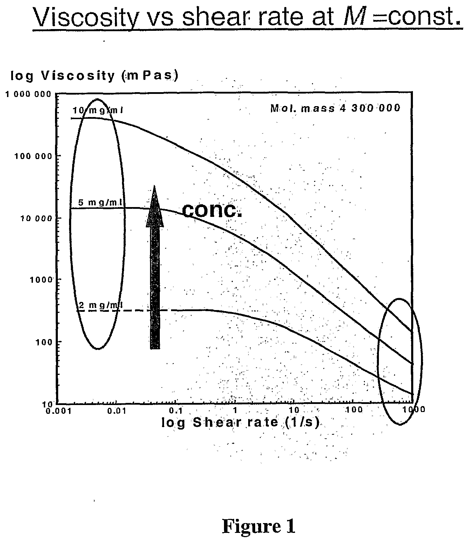

**Figure 1** shows the rheological properties of three monomodal solutions of sodium hyaluronate having a mol. mass of 4,300,000, the solutions varying in concentration from 2 mg/ml to 10 mg/ml. As can be seen, the concentration of the hyaluronate also significantly impacts the rheological properties of the solutions.

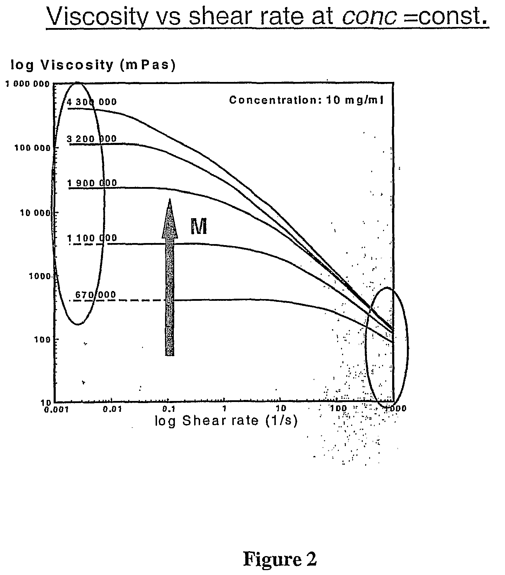

**Figure 2** shows the rheological properties of five monomodal solutions of sodium hyaluronate of varying molecular mass, but constant concentration. As can be seen, the molecular mass of the hyaluronate significantly impacts the rheological properties of the solutions.

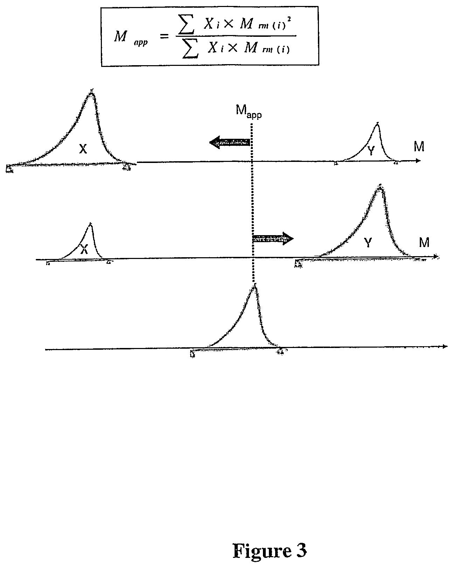

**Figure 3** is a conceptual mapping, showing that it is possible to construct a solution having an apparent molecular mass ($M_{app}$) in an infinite number of ways, by changing the proportions and the average molecular mass of each distribution. The rheological properties of the final solution, whether a bi-modal (or multi-modal), will depend on these choices and on the total concentration of the viscoelastic used. Formulations can be modified to broaden the molecular mass distribution and content of HA or other polymer in different areas of the distribution using different polymer fractions.

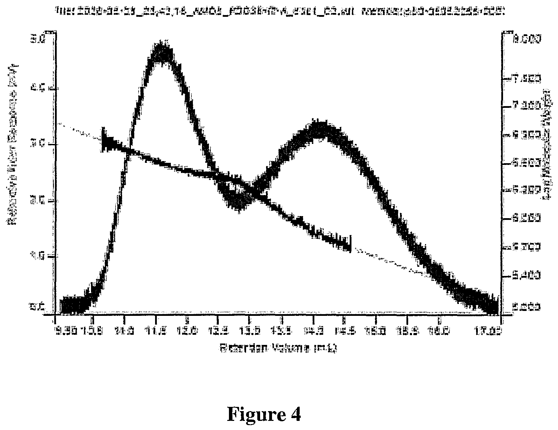

**Figure 4** shows the molecular mass distribution of a bimodal formulation as determined using GPC chromatography coupled with LALLS detection. If the proportion between the high and low molecular mass is changed, it is possible to manipulate/choose the Mapp. Thus, the performance of the solution can be controlled by where the mean of the two populations are and the % composition of each component. By having distinct distributions, it is possible to maximize the benefits of the high and low molecular mass components.

## Table 1

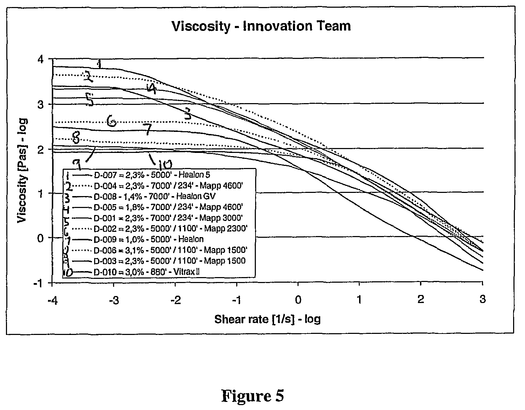

The compositions of the various solutions ($M_{rm}$'s given are pre-autoclave) noted in **Figure 5** are shown in Table 1. The solutions D-002, D-003 and D-006 are not according to the invention. D-007, D-008, D-009 and D-010 are also not according to the invention and are provided for information only.

**Table 1**

| Solution No. | Conc. HA | $M_{rm}$ of High MW Fraction | $M_{rm}$ of Low $M_{rm}$ Fraction | $M_{app}$ |
|---|---|---|---|---|
| D-001 | 2.3% | 7,000,000 | 234,000 | 3,000,000 |
| D-002 | 2.3% | 5,000,000 | 1,100,000 | 2,300,000 |
| D-003 | 2.3% | 5,000,000 | 1,100,000 | 1,500,000 |
| D-004 | 2.3% | 7,000,000 | 234,000 | 4,600,000 |
| D-005 | 1.8% | 7,000,000 | 234,000 | 4,600,000 |
| D-006 | 3.1% | 5,000,000 | 1,100,000 | 1,500,000 |
| D-007 (HEALON 5)) | 2.3% | 5,000,000 | N/A | N/A |

(continued)

| Solution No. | Conc. HA | $M_{rm}$ of High MW Fraction | $M_{rm}$ of Low $M_{rm}$ Fraction | $M_{app}$ |
|---|---|---|---|---|
| D-008 (HEALON GV) | 1.4% | 7,000,000 | N/A | N/A |
| D-009 (HEALON) | 1.0% | 5,000,000 | N/A | N/A |
| D-010 (VITRAX II) | 3.0% | 880,000 | N/A | N/A |

As can be seen, the control of the concentration and molecular mass of the different fractions significantly affects the performance of the solution. Thus, the performance of the solution can be controlled by where the molecular mass mean of the two populations and the % composition of each component are. Thus, it is possible to maximize the benefits of the high and low molecular mass components.

It is noted that the $M_{rm}$ of the above compositions are given in pre-autoclave molecular mass, while some of the data is obtained from solutions that have been autoclaved. It is well known that autoclaving has a greater effect on the higher molecular mass polymers. One of ordinary skill in the art will understand that autoclaving has a tendency to break down the polymer to a certain extent, and will be able to calculate or readily determine either (a) the post-autoclaving molecular mass of the solutions as well as their components, or (b) the pre-autoclaving solution(s) which would produce the solutions given herein, sterilized.

While the solution for use in ophthalmic surgery of the present invention has been generally described as particularly suited for ophthalmic surgery, one of ordinary skill in the art will realize that the solution itself may also be utilized in the fields of viscoelastic supplementation (for example, joint administration), cosmetics/plastics (for example, as a dermal filler), and wound healing (for example, as an anti-adhesion agent).

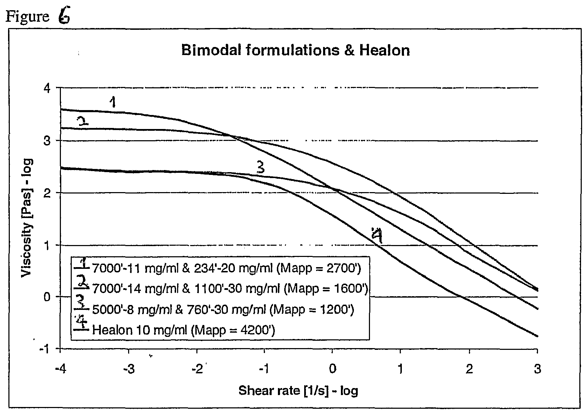

The data shown in **Figure 6** demonstrates that it is possible to control the shear viscosity at different shear rates of importance for different surgical steps of the various solutions according to the present invention. As can be seen, the two bottom curves (labeled 3 and 4) have the same zero shear viscosity, but they are formulated differently. Number 4 is standard mono modal Healon OVD and the bi-modal, number 3, is formulated using a relatively high molecular mass HA, (5 million) at 8 mg/ml, and a relatively low molecular mass (760,000) HA at 30 mg/ml. Number 4 does not form part of the present invention and is provided for information only. The total concentration of the solution number 3 is thus 38 mg/ml and the $M_{app}$ is 1.2 million. These differences are reflected in a higher viscosity on the high shear rate side compared to the standard Healon OVD, which has a concentration of only 10 mg/ml. Both of these formulations have the same space maintaining capability (zero shear viscosity) but with very different flow properties (viscosities at higher shear rates).

Curve number 2 has a higher molecular mass of the high component (7 million, 14 mg/ml) and the "low" mass (1.1 million, 30 mg/ml) is slightly higher than that in the "red curve". This leads to a slightly higher $M_{app}$, (1.6 million) which together with the total concentration 44 mg/ml is reflected on the "low" shear side by a higher shear viscosity in comparison to curve number 3. It should be noted that curve number 2 and curve number 3 start at very different zero shear viscosities. However, due to their similar concentrations, they end up with similar viscosities on the high shear rate side.

Curve number 1, a bi-modal curve having in this comparison the highest $M_{app}$, of the bi-modals and a total concentration of 31 mg/ml. The total concentration of HA gives this formulation flow properties on the high shear rate side that is in between the Healon OVD (10 mg/ml) and for example the formulation (44 mg/ml) shown with curve number 2. On the low shear rate side where the viscosity is dependent on the concentration multiplied with molecular mass, the decrease in viscosity is steeper due firstly to the high $M_{app}$, and secondly to the lower total concentration of HA compared to that in curve number 2.

As is demonstrated by the data presented in the above table the entire shear viscosity curve can be controlled to meet the clinical needs by changing (a) the total concentration of HA, (b) the apparent Molecular mass $M_{app}$, and (c) the proportions of the different distributions of HA substances with different $M_{rm}$.

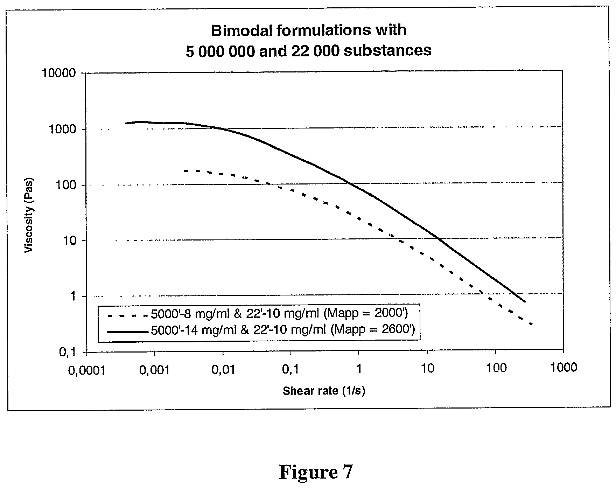

**Figure 7** shows two bi-modal formulations made of a comparatively high molecular mass HA and a very low $M_{rm}=22\ 000$. While these solutions have not too different $M_{app}$'s , their zero shear viscosities are quite different also due to higher total HA concentration of the upper, solid-line curve.

One of the benefits of the present invention is that it is possible to utilize the benefits of both the high and low molecular mass constituents of the composition. That is, the wettability of a viscoelastic formulation as measured by contact angle on a hydrophilic surface may be changed. Table 2 compares 4 solutions. Each has the same concentration of a high molecular mass and low molecular mass fraction. The main difference between the solutions is the molecular mass of the lower mass fraction, and the resulting contact angle. The contact angle associated with the solutions having the lower, lower molecular mass components is lower than the contact angle associated with the higher, lower molecular mass component. Since a lower contact angle is associated with a greater ability to coat/protect tissues, it is expected that solution 1 would provide a greater coating ability.

**Table 2**

| Solution No. | Total Amount HA (mg/ml) | Amount high comp. (mg/ml) | Amount low comp. (mg/ml) | High comp. | low comp. | Contact angle |
|---|---|---|---|---|---|---|
| 1 | 18 mg/ml | 8 | 10 | 5000' | 22' | **23.9** |
| 2 | 18 mg/ml | 8 | 10 | 5000' | 234' | **27.1** |
| 3 | 18 mg/ml | 8 | 10 | 5000' | 760' | **30.0** |
| 4* | 18 mg/ml | 8 | 10 | 5000' | 1100' | **34.6** |
| *Solution No. 4 does not form part of the present invention and is provided for information only. | | | | | | |

As may also be seen from the data presented in Table 2, an increase in the difference in molecular mass between the two polymer fractions leads to an accentuation of the desirable properties of the OVD. For example, when comparing solutions 4 and 3, which have a difference of 3.9 million and 4.24 million, respectively, it is possible to see that the higher difference in molecular mass leads to a lower contact angle, and hence greater wetting or coating ability. The same may be said of solution 2, which has a difference in molecular mass of 4.766 and solution 1, which as a difference in molecular mass of almost 5 million. In the present invention, the difference in molecular mass is greater than 4 million. In an alternate embodiment, the difference is greater than 4.5 million, or greater than 5 million. In a still further embodiment, the difference is greater than 6 million.

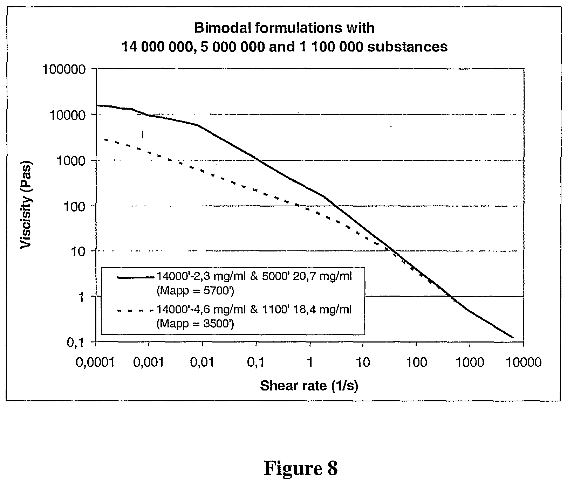

**Figure 8** shows two bi-modal formulations of a super high $M_{rm}$ 14,000 with two different "low" HA components. The total concentration is 23 mg/ml in both cases, therefore the viscosity on the high shear side is the same. The difference on the low shear rate side is thus due to the different $M_{app}$.

While the foregoing is a complete description of the preferred embodiments of the invention, various alternatives, modifications, and equivalents can be used. Moreover, it will be obvious that certain other modifications can be practiced within the scope of the appended claims.

**Claims**

1. A relatively homogeneous solution for use in ophthalmic surgery, the solution comprising a first fraction of a first ophthalmically acceptable compound and a second fraction of a second ophthalmically acceptable compound, wherein the first and second fractions are present in a total concentration of at least 10 mg/ml, and the first and second fractions have average molecular mass that differ by at least 4 million, wherein each of the first and second compounds comprises a hyaluronate.

2. The relatively homogeneous solution for use according to claim 1, wherein the first compound has an average molecular mass of at least 5 million.

3. The relatively homogeneous solution for use according to claim 1, wherein the second compound has an average molecular mass of less than 1.5 million.

4. The relatively homogeneous solution for use according to claim 1, wherein the first compound has an average molecular mass of at least 5 million and the second compound has an average molecular mass of at least 0.75 million.

5. The relatively homogeneous solution for use according to claim 1, wherein the first and second fractions have average molecular mass that differ by at least five million.

6. The relatively homogeneous solution for use according to claim 1, further comprising a third fraction of a third ophthalmically acceptable compound, present in a concentration of at least 5 mg/ml, and wherein the average molecular mass of the third fraction differs from the average molecular mass of at least one of the first and second fractions by at least one million.

**Patentansprüche**

1. Relativ homogene Lösung zur Verwendung in der Augenchirurgie, wobei die Lösung eine erste Fraktion einer ersten ophthalmisch unbedenklichen Verbindung und eine zweite Fraktion einer zweiten ophthalmisch unbedenklichen Verbindung umfasst, wobei die erste und die zweite Fraktion in einer Gesamtkonzentration von mindestens 10 mg/ml vorliegen und die erste und die zweite Fraktion durchschnittliche Molekülmassen haben, die sich um mindestens 4 Millionen unterscheiden, wobei die erste und die zweite Verbindung jeweils ein Hyaluronat umfassen.

2. Relativ homogene Lösung zur Verwendung nach Anspruch 1, wobei die erste Verbindung eine durchschnittliche Molekülmasse von mindestens 5 Millionen hat.

3. Relativ homogene Lösung zur Verwendung nach Anspruch 1, wobei die zweite Verbindung eine durchschnittliche Molekülmasse von weniger als 1,5 Millionen hat.

4. Relativ homogene Lösung zur Verwendung nach Anspruch 1, wobei die erste Verbindung eine durchschnittliche Molekülmasse von mindestens 5 Millionen hat und die zweite Verbindung eine durchschnittliche Molekülmasse von mindestens 0,75 Millionen hat.

5. Relativ homogene Lösung zur Verwendung nach Anspruch 1, wobei die erste und die zweite Fraktion durchschnittliche Molekülmassen haben, die sich um mindestens 5 Millionen unterscheiden.

6. Relativ homogene Lösung zur Verwendung nach Anspruch 1, welche weiterhin eine dritte Fraktion einer dritten ophthalmisch unbedenklichen Verbindung umfasst, die in einer Konzentration von mindestens 5 mg/ml vorliegt, und wobei die durchschnittliche Molekülmasse der dritten Fraktion sich von der durchschnittlichen Molekülmasse mindestens einer der ersten und der zweiten Fraktion um mindestens eine Million unterscheidet.

**Revendications**

1. Solution relativement homogène pour utilisation en chirurgie ophtalmique, la solution comprenant une première fraction d'un premier composé acceptable sur le plan ophtalmique et une deuxième fraction d'un deuxième composé acceptable sur le plan ophtalmique, dans laquelle les première et deuxième fractions sont présentes à une concentration totale d'au moins 10 mg/ml, et dans laquelle les première et deuxième fractions ont une masse moléculaire moyenne qui diffère d'au moins 4 millions, chacun des premier et deuxième composés comprenant un hyaluronate.

2. Solution relativement homogène pour utilisation selon la revendication 1, dans laquelle le premier composé a une masse moléculaire moyenne d'au moins 5 millions.

3. Solution relativement homogène pour utilisation selon la revendication 1, dans laquelle le deuxième composé a une masse moléculaire moyenne d'au moins 1,5 million.

4. Solution relativement homogène pour utilisation selon la revendication 1, dans laquelle le premier composé a une masse moléculaire moyenne d'au moins 5 millions et le deuxième composé a une masse moléculaire moyenne d'au moins 0,75 million.

5. Solution relativement homogène pour utilisation selon la revendication 1, dans laquelle les première et deuxième fractions ont une masse moléculaire moyenne qui diffère d'au moins cinq millions.

6. Solution relativement homogène pour utilisation selon la revendication 1, comprenant en outre une troisième fraction d'un troisième composé acceptable sur le plan ophtalmique, présent à une concentration d'au moins 5 mg/ml, et dans laquelle la masse moléculaire moyenne de la troisième fraction diffère de la masse moléculaire moyenne d'au moins une des première et deuxième fractions d'au moins un million.

## <u>Viscosity vs shear rate at $M$ =const.</u>

**Figure 1**

## Viscosity vs shear rate at *conc* =const.

Figure 2

$$M_{app} = \frac{\sum X_i \times M_{rm(i)}^2}{\sum X_i \times M_{rm(i)}}$$

Figure 3

**Figure 4**

**Figure 5**

Figure 6

**Bimodal formulations & Healon**

Legend (inset):
1. 7000'-11 mg/ml & 234'-20 mg/ml (Mapp = 2700')
2. 7000'-14 mg/ml & 1100'-30 mg/ml (Mapp = 1600')
3. 5000'-8 mg/ml & 760'-30 mg/ml (Mapp = 1200')
4. Healon 10 mg/ml (Mapp = 4200')

X-axis: Shear rate [1/s] - log
Y-axis: Viscosity [Pas] - log

**Figure 7**

Figure 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4141973 A
- US 4303676 A
- US 6107347 A, Francese
- US 5492936 A